# EUROPEAN PATENT APPLICATION

(11) **EP 3 017 878 A1**
(43) Date of publication of application: **11.05.2016**
(21) Application number: 14819931.8
(22) Date of filing: 26.03.2014
(51) Int. Cl.: B06B 1/06, A61B 18/00, H04R 17/00

(54) **ULTRASONIC VIBRATION DEVICE, ULTRASONIC VIBRATION DEVICE MANUFACTURING METHOD, AND ULTRASONIC MEDICAL APPARATUS**

(30) Priority: 03.07.2013 JP 2013139890
(71) Applicant: Olympus Corporation, Shibuya-ku Tokyo 151-0072 (JP)
(72) Inventor: SAKAI Nagahide, Tokyo 151-0072 (JP); JINBO, Hikaru, Tokyo 151-0072 (JP); ITO, Hiroshi, Tokyo 151-0072 (JP)
(74) Representative: Winter, Brandl, Fürniss, Hübner, Röss, Kaiser, Polte - Partnerschaft mbB
(86) International application number: PCT/JP2014/058425
(87) International publication number: WO 2015/001822

(57) **Abstract**

An ultrasound vibration device 2 includes a stacked transducer 41 in which a plurality of piezoelectric substances 61 and a plurality of electrode plates 62 and 63 are stacked, an external shape of the stacked transducer 41 being formed in a polygonal prism shape, insulators 42 and 43 disposed at both ends of the stacked transducer 41, a case main body 52 that houses the piezoelectric transducer 61 and the insulators 42 and 43, a pressurizing member 51 screwed and fastened to an opening portion of the case main body 52 to pressurize and fix the stacked transducer 41 and the insulators 42 and 43 in the case main body 52, and a plurality of positioning-member insertion sections 54 into which a positioning member 100 is inserted, the positioning member 100 retaining, in the case main body 52, the stacked transducer 41 and the insulators 42 and 43 in the polygonal prism shape and positioning the stacked transducer 41 in a position where the stacked transducer 41 is not in contact with an inner wall of the case main body 53 and one ends of the insulators 42 and 43 are in contact with the pressurizing member 51.

## Description

### Technical Field

The present invention relates to an ultrasound vibration device that excites ultrasound vibration, a manufacturing method for the ultrasound vibration device, and an ultrasound medical apparatus including the ultrasound vibration device.

### Background Art

As an ultrasound treatment instrument that performs coagulation/dissection treatment of a biological tissue using ultrasound vibration, there is an ultrasound treatment instrument incorporating a Langevin type transducer in a hand piece as an ultrasound vibration source.

Such a Langevin type transducer is disclosed in, for example, Patent Literature 1. In the Langevin type transducer disclosed in Patent Literature 1, there has been proposed a technique for stacking and housing a plurality of piezoelectric elements in a vibration block and preventing a short circuit due to, for example, dirts of the piezoelectric elements.

### Citation List

### Patent Literature

Patent Literature 1: Japanese Patent Application Laid-Open Publication No. 2003-199195

### Summary of Invention

### Technical Problem

However, a shape of the piezoelectric elements disclosed in Patent Literature 1 is processed in a disk shape. Therefore, there is a disadvantage that, when the piezoelectric elements are cut out in the disk shape from a piezoelectric material wafer, the number of piezoelectric elements taken from the piezoelectric material wafer is small. Therefore, it is desirable to cut out the piezoelectric elements in a rectangular shape such that a large number of the piezoelectric elements can be taken.

When piezoelectric monocrystal chips cut out in the rectangular shape in this way are stacked to assemble the Langevin type transducer, in the technique of Patent Literature 1, if a plurality of the piezoelectric elements are stacked in the vibration block, position accuracy and stacking accuracy of the piezoelectric elements are determined only by electrode pieces that are in contact with the stacked piezoelectric elements. Therefore, it is extremely difficult to perform accurate positioning of the plurality of piezoelectric elements. Further, it is likely that a vibrator side surface comes into contact with a vibration block inner wall because of fastening in the assembly.

That is, in the technique for stacking the plurality of piezoelectric elements in the vibration block as in the past like Patent Literature 1, there is a concern about occurrence of deficiencies such as an output decrease, abrasion powder occurrence, and a short-circuit failure due to vibration attenuation.

Therefore, the present invention has been devised in view of the problems and it is an object of the present invention to make it possible to provide an ultrasound vibration device, a manufacturing method for the ultrasound vibration device, and an ultrasound medical apparatus that makes it possible to improve productivity and accurately position and stack piezoelectric elements without causing the piezoelectric elements to interfere with a case to prevent deficiencies such as an output decrease, abrasion powder occurrence, and a short-circuit failure due to vibration attenuation.

### Disclosure of the invention

### Solution to Problem

An ultrasound vibration device according to an aspect of the present invention includes: a stacked transducer in which a plurality of piezoelectric substances and a plurality of electrode plates are stacked, an external shape of the stacked transducer being formed in a polygonal prism shape; insulators disposed at both ends of the stacked transducer; a case main body that houses the piezoelectric transducer and the insulators; a pressurizing member screwed and fastened to an opening portion of the case main body to pressurize and fix the stacked transducer and the insulators in the case main body; and a plurality of positioning member insertion sections into which a positioning member is inserted, the positioning member retaining, in the case main body, the stacked transducer and the insulators in the polygonal prism shape and positioning the stacked transducer in a position where the stacked transducer is not in contact with an inner wall of the case main body and one ends of the insulators are in contact with the pressurizing member.

A manufacturing method for an ultrasound vibration device according to an aspect of the present invention is a manufacturing method for an ultrasound vibration device including: a stacked transducer in which a plurality of piezoelectric substances and a plurality of electrode plates are stacked, an external shape of the stacked transducer being formed in a polygonal prism shape; insulators disposed at both ends of the stacked transducer; a case main body that houses the piezoelectric transducer and the insulators; a pressurizing member screwed and fastened to an opening portion of the case main body to pressurize and fix the stacked transducer and the insulators in the case main body; and a plurality of positioning member insertion sections into which a positioning member is inserted, the positioning member retaining, in the case main body, the stacked transducer and the insulators in the polygonal prism shape and positioning the stacked transducer in a position where the stacked transducer is not in contact with an inner wall of the case main body and one ends of the insulators are in contact with the pressurizing member. The manufacturing method for the ultrasound vibration device including: housing the stacked transducer, at both the ends of which the insulators are disposed, in a position in non-contact with the inner wall of the case main body; inserting a plurality of the positioning members having V grooves formed at distal ends into the plurality of positioning member insertion sections of the case main body, bringing the V grooves of the positioning members into contact with opposed corner portions of a stacked body formed by the insulator and the stacked transducer, and positioning the stacked body in a state in which the stacked body is retained in the polygonal prism shape in the case main body; screwing and fastening the pressurizing member to the case main body and pressurizing and fixing the stacked body with the pressurizing member and a bottom section of the case main body; and retracting and removing the plurality of positioning members from the positioning member insertion sections.

Further, an ultrasound medical apparatus according to an aspect of the present invention includes: an ultrasound vibration device including: a stacked transducer in which a plurality of piezoelectric substances and a plurality of electrode plates are stacked, an external shape of the stacked transducer being formed in a polygonal prism shape; insulators disposed at both ends of the stacked transducer; a case main body that houses the piezoelectric transducer and the insulators; a pressurizing member screwed and fastened to an opening portion of the case main body to pressurize and fix the stacked transducer and the insulators in the case main body; and a plurality of positioning member insertion sections into which a positioning member is inserted, the positioning member retaining, in the case main body, the stacked transducer and the insulators in the polygonal prism shape and positioning the stacked transducer in a position where the stacked transducer is not in contact with an inner wall of the case main body and one ends of the insulators are in contact with the pressurizing member; and a probe distal end portion to which ultrasound vibration generated by the ultrasound vibration device is transmitted, the probe distal end portion treating a biological tissue.

According to the present invention described above, it is possible to provide an ultrasound vibration device, a manufacturing method for the ultrasound vibration device, and an ultrasound medical apparatus that makes it possible to improve productivity and accurately position and stack piezoelectric elements without causing the piezoelectric elements to interfere with a case to prevent deficiencies such as an output decrease, abrasion powder occurrence, and a short-circuit failure due to vibration attenuation.

### Brief Description of the Drawings

Fig. 1 is a cross-sectional view showing an overall configuration of an ultrasound medical apparatus according to a first embodiment of the present invention;
Fig. 2 is a diagram showing a schematic configuration of an entire transducer unit according to the first embodiment;
Fig. 3 is a perspective view showing a configuration of the transducer unit according to the first embodiment;
Fig. 4 is an exploded perspective view showing a configuration of a stacked body formed by insulators, rectangular piezoelectric substances, and electrode plates according to the first embodiment;
Fig. 5 is an exploded perspective view showing a configuration of the transducer unit according to the first embodiment;
Fig. 6 is an exploded perspective view showing an assembled state of an ultrasound transducer according to the first embodiment;
Fig. 7 is a cross-sectional view showing the assembled state of the ultrasound transducer according to the first embodiment;
Fig. 8 is a perspective view showing a configuration of a case main body of a modification of the first embodiment;
Fig. 9 is a perspective view showing a configuration of a case main body of a modification of a form different from Fig. 8 in the first embodiment;
Fig. 10 is a cross-sectional view showing an assembled state of an ultrasound transducer of the modifications shown in Fig. 8 and Fig. 9 in the first embodiment;
Fig. 11 is a perspective view showing a configuration of a case main body according to a second embodiment of the present invention;
Fig. 12 is a diagram showing a state in which a stacked transducer is housed in a case main body and held by a positioning member according to the second embodiment;
Fig. 13 is a cross-sectional view showing an assembled state of an ultrasound transducer according to the second embodiment;
Fig. 14 is an exploded perspective view showing an assembled state of an ultrasound transducer of a first modification of the second embodiment;
Fig. 15 is an exploded perspective view showing an assembled state of an ultrasound transducer of a second modification of the second embodiment;
Fig. 16 is a partial sectional view of the ultrasound transducer of the second modification of the second embodiment;
Fig. 17 is a partial sectional view of a state in which a stacked body formed by insulators, rectangular piezoelectric substances, and electrode plates is positioned by positioning members in a case and pressurized by a lid according to a third embodiment of the present invention;
Fig. 18 is a partial sectional view of a state in which the stacked body in which tolerances of plus tendency occurs in the insulators, the rectangular piezoelectric substances, and the electrode plates is positioned by the positioning members in the case and pressurized by the lid according to the third embodiment;
Fig. 19 is a perspective view showing a configuration of a lid according to a first aspect of the third embodiment;
Fig. 20 is a partial sectional view of a state in which a stacked body formed by insulators, rectangular piezoelectric substances, and electrode plates is positioned by positioning members in a case and pressurized by a lid according to the first aspect of the third embodiment;
Fig. 21 is a partial sectional view of a state in which the stacked body in which tolerances of minus tendency occurs in the insulators, the rectangular piezoelectric substances, and the electrode plates is positioned by the positioning members in the case and pressurized by the lid according to the third embodiment;
Fig. 22 is a perspective view showing a configuration of a lid according to a second aspect of the third embodiment;
Fig. 23 is a partial sectional view of a state in which a stacked body formed by insulators, rectangular piezoelectric substances, and electrode plates is positioned by positioning members in a case and pressurized by a lid according to the second aspect of the third embodiment;
Fig. 24 is a perspective view showing a configuration of a lid according to a third aspect of the third embodiment;
Fig. 25 is a partial sectional view of a state in which a stacked body formed by insulators, rectangular piezoelectric substances, and electrode plates is positioned by positioning members in a case and pressurized by a lid according to the third aspect of the third embodiment;
Fig. 26 is an exploded perspective view showing a configuration of an ultrasound transducer according to a fourth aspect of the third embodiment;
Fig. 27 is an exploded perspective view showing an assembled state of the ultrasound transducer according to the fourth aspect of the third embodiment;
Fig. 28 is a partial sectional view of a state in which a stacked body formed by insulators, rectangular piezoelectric substances, and electrode plates is positioned by positioning members in a case and pressurized by a lid according to the fourth aspect of the third embodiment; and
Fig. 29 is a cross-sectional view of the ultrasound transducer taken along line XXIX-XXIX in Fig. 28.

### Best Mode for Carrying Out the Invention

The present invention is explained below with reference to the drawings.

Note that, in the following explanation, it should be noted that the drawings based on respective embodiments are schematic and relations between thicknesses and widths of respective portions, ratios of the thicknesses of the respective portions, and the like are different from real ones. Portions, relations and ratios of dimensions of which are different from one another among the drawings, are sometimes included.

### (First Embodiment)

First, a first embodiment of the present invention is explained below with reference to the drawings.

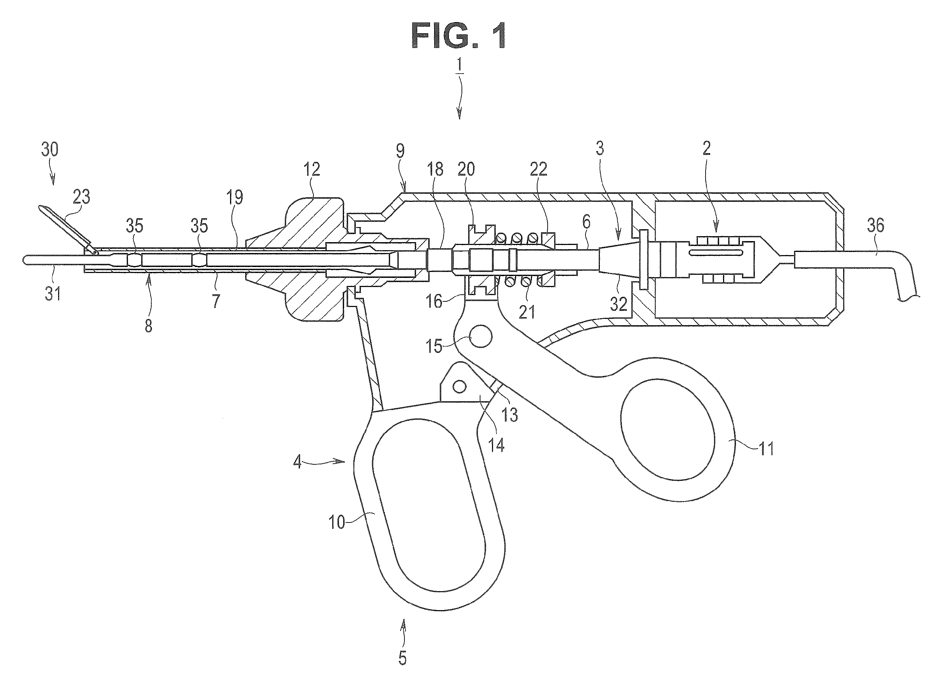

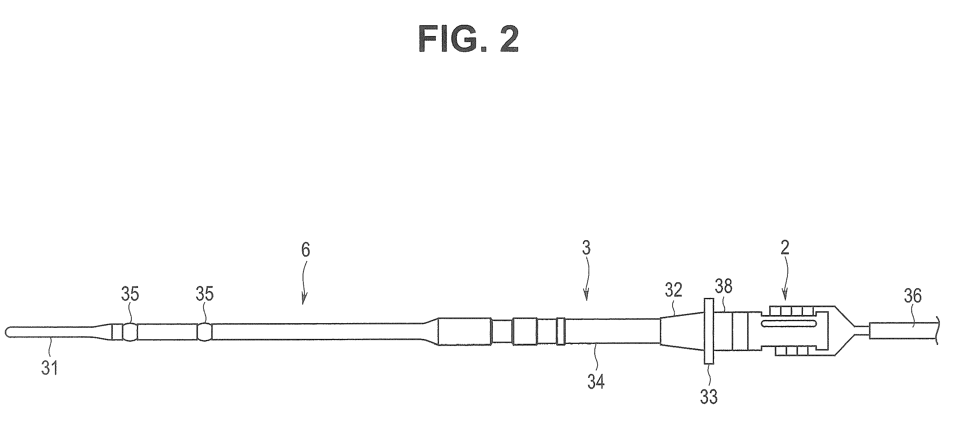

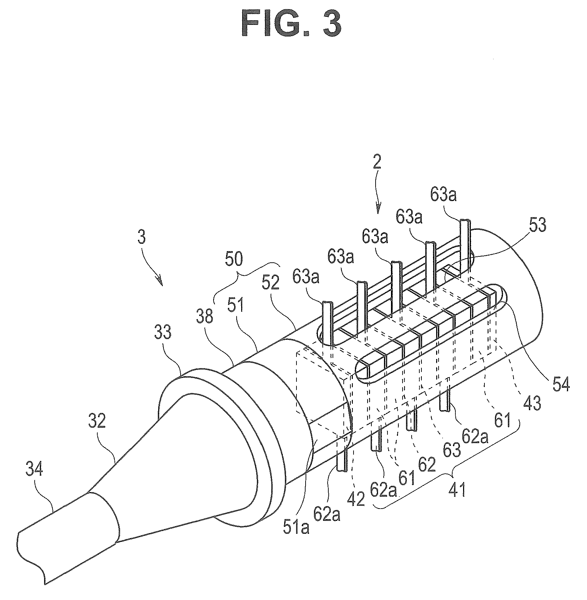

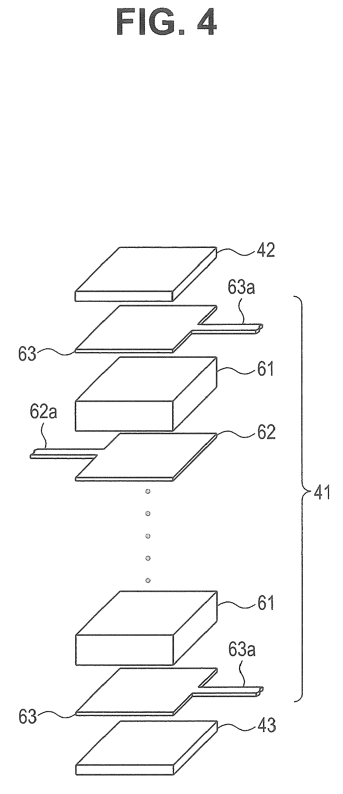

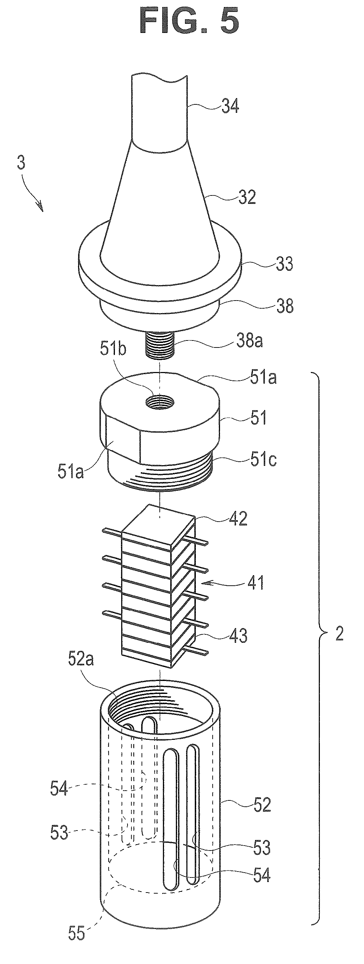

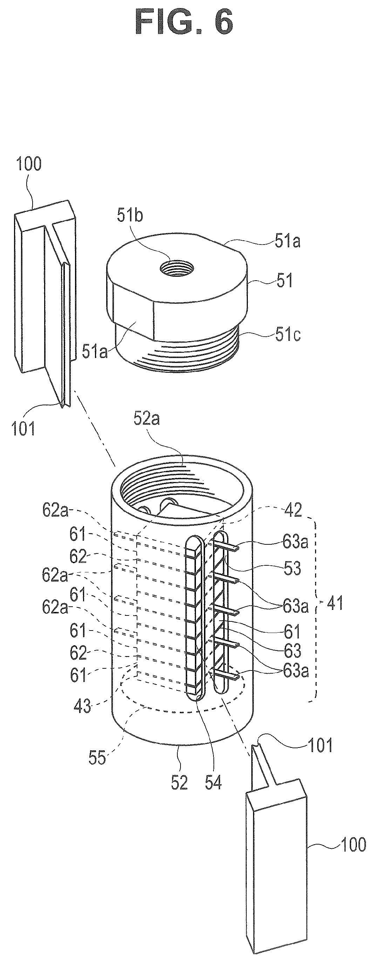

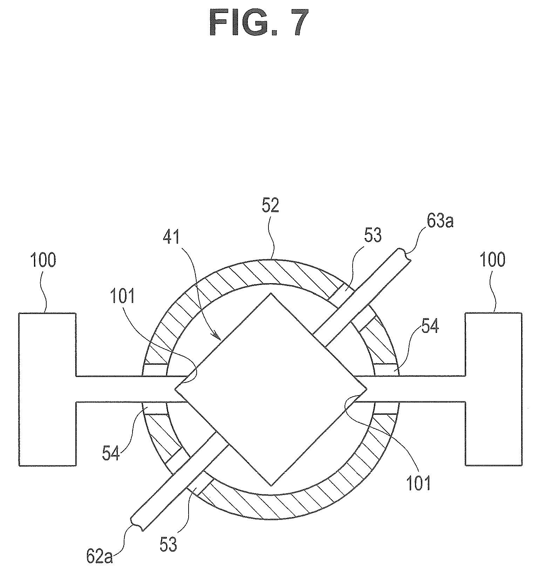

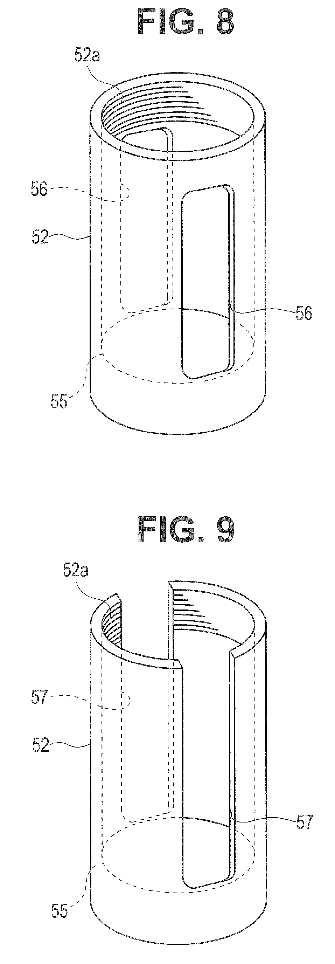

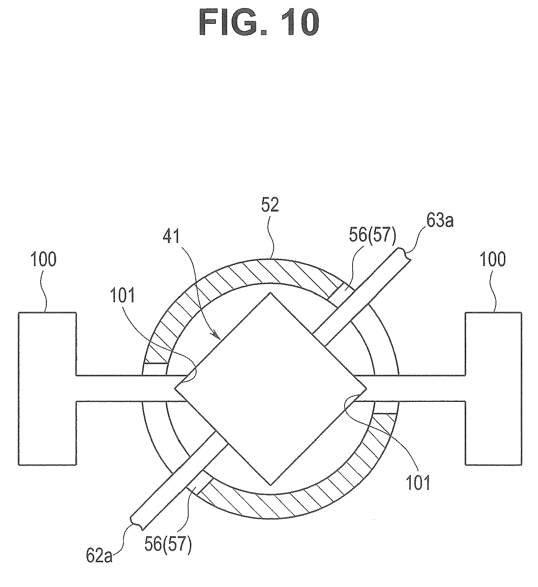

Fig. 1 is a cross-sectional view showing an overall configuration of an ultrasound medical apparatus. Fig. 2 is a diagram showing a schematic configuration of an entire transducer unit. Fig. 3 is a perspective view showing a configuration of the transducer unit. Fig. 4 is an exploded perspective view showing a configuration of a stacked body formed by insulators, rectangular piezoelectric substances, and electrode plates. Fig. 5 is an exploded perspective view showing a configuration of the transducer unit. Fig. 6 is an exploded perspective view showing an assembled state of an ultrasound transducer. Fig. 7 is a cross-sectional view showing the assembled state of the ultrasound transducer. Fig. 8 is a perspective view showing a configuration of a case main body of a modification. Fig. 9 is a perspective view showing a configuration of a case main body of a modification of a form different from Fig. 8. Fig. 10 is a cross-sectional view showing an assembled state of an ultrasound transducer of the modifications shown in Fig. 8 and Fig. 9.

### (Ultrasound medical apparatus)

An ultrasound medical apparatus 1 shown in Fig. 1 is mainly provided with a transducer unit 3 including an ultrasound transducer 2 functioning as an ultrasound device that generates ultrasound vibration and a handle 4 that performs treatment of a diseased part using the ultrasound vibration.

The handle 4 includes an operation section 5, an insertion sheath 8 formed by a long mantle tube 7, and a distal-end treatment section 30. A proximal end portion to the insertion sheath 8 is attached rotatably in a direction around an axis of the operation section 5. The distal-end treatment section 30 is provided at a distal end of the insertion sheath 8. The operation section 5 of the handle 4 includes an operation section main body 9, a fixed handle 10, a movable handle 11, and a rotation knob 12. The operation section main body 9 is formed integrally with the fixed handle 10.

A slit 13, through which the movable handle 11 is inserted, is formed on a back side in a coupling section of the operation section main body 9 and the fixed handle 10. An upper part of the movable handle 11 is extended to an inside of the operation section main body 9 through the slit 13. A handle stopper 14 is fixed to an end portion on a lower side of the slit 13. The movable handle 11 is turnably attached to the operation section main body 9 via a handle support shaft 15. According to a motion of the movable handle 11 turning centering on the handle support shaft 15, the movable handle 11 is opened and closed with respect to the fixed handle 10.

A substantially U-shaped coupling arm 16 is provided in an upper end portion of the movable handle 11. The insertion sheath 8 includes the mantle tube 7 and an operation pipe 19 inserted through the mantle tube 7 movably in an axial direction. A large diameter section 18 larger in diameter than a distal end side portion is formed at a proximal end portion of the mantle tube 7. The rotation knob 12 is attached around the large diameter section 18.

On an outer circumferential surface of the operation pipe 19, a ring-like slider 20 is movably provided along the axial direction. A fixed ring 22 is disposed behind the slider 20 via a coil spring (an elastic member) 21.

Further, a proximal end portion of a grasping section 23 is turnably coupled to a distal end portion of the operation pipe 19 via an action pin. The grasping section 23 configures a treatment section of the ultrasound medical apparatus 1 in conjunction with a distal end portion 31 of a probe 6. During a motion of the operation pipe 19 moving in the axial direction, the grasping section 23 is pushed and pulled in a front-back direction via the action pin. At this point, during a motion of the operation pipe 19 moved to a hand side, the grasping section 23 is turned around a fulcrum pin via the action pin. Consequently, the grasping section 23 turns in a direction in which the grasping section 23 approaches the distal end portion 31 of the probe 6 (a closing direction). At this point, a biological tissue can be grasped between the grasping section 23 of a single swing type and the distal end portion 31 of the probe 6.

In a state in which the biological tissue is grasped in this way, electric power is supplied from an ultrasound power supply to the ultrasound transducer 2 to vibrate the ultrasound transducer 2. The ultrasound vibration is transmitted to the distal end portion 31 of the probe 6. Treatment of the biological tissue grasped between the grasping section 23 and the distal end portion 31 of the probe 6 is performed using the ultrasound vibration.

### (Transducer unit)

Here, the transducer unit 3 is explained.

The transducer unit 3 is a unit in which, as shown in Fig. 2, the ultrasound transducer 2 and the probe 6, which is a bar-like vibration transmission member that transmits ultrasound vibration generated in the ultrasound transducer 2, are integrally assembled.

A horn 32 that amplifies amplitude is concatenated to the ultrasound transducer 2. The horn 32 is shaped by duralumin or a titanium alloy such as 64Ti. The horn 32 is shaped in a conical shape reduced in an outer diameter toward a distal end side. An outward flange 33 for securing to the operation section main body 9 (see Fig. 1) is shaped in a halfway outer circumferential section. The horn 32 comprises a proximal end columnar section 38 behind the outward flange 33.

The probe 6 comprises a probe main body 34 made of a titanium alloy such as 64Ti. The ultrasound transducer 2 concatenated to the aforementioned horn 32 is disposed on a proximal end portion side of the probe main body 34. In this way, the transducer unit 3 in which the probe 6 and the ultrasound transducer 2 are integrated is formed.

The ultrasound vibration generated in the ultrasound transducer 2 is amplified by the horn 32 and thereafter transmitted to the distal end portion 31 side of the probe 6. In the distal end portion 31 of the probe 6, a treatment section explained below that treats a biological tissue is formed.

Two rubber linings 35 made of an elastic member in a ring shape are attached to an outer circumferential surface of the probe main body 34 at intervals in several parts of node positions of vibration halfway in the axial direction. Contact of the outer circumferential surface of the probe main body 34 and the operation pipe 19 explained below is prevented by the rubber linings 35.

That is, when the insertion sheath 8 is assembled, the probe 6 functioning as a transducer-integrated probe is inserted into an inside of the operation pipe 19. At this point, the contact of the outer circumferential surface of the probe main body 34 and the operation pipe 19 is prevented by the rubber linings 35.

Note that the ultrasound transducer 2 is electrically connected to, via an electric cable 36, a not-shown power supply device main body that supplies an electric current for generating ultrasound vibration. Electric power is supplied from a power supply device main body of an external apparatus to the ultrasound transducer 2 through a wire in the electric cable 36, whereby the ultrasound transducer 2 is driven.

### (Ultrasound transducer)

The ultrasound transducer 2 functioning as a stacked ultrasound vibration device of the present invention is explained below.

In the ultrasound transducer 2 of the transducer unit 3, as shown in Fig. 3, a stacked transducer 41 stacked in a rectangular shape (a square pole shape) is incorporated in a case 50 concatenated to the proximal end columnar section 38 behind the horn 32.

In the stacked transducer 41, as shown in Fig. 4, rectangular piezoelectric substances 61 formed in a polygonal shape, here, a rectangular shape are stacked. In the stacked transducer 41, insulators 42 and 43 formed of ceramics or the like in a polygonal shape, here, a rectangular shape are disposed on both end sides. The stacked transducer 41 is sandwiched by the two insulators 42 and 43 horizontally (vertically on a paper surface; in the following explanation, vertically on the paper surface is sometimes referred to as horizontally).

Incidentally, as the rectangular piezoelectric substances 61 of the present embodiment, a piezoelectric material such as lead zirconate titanate (PZT, Pb(Zrx, Ti₁₋ₓ)O3) or lithium niobate single crystal (LiNbO3) of a piezoelectric single crystal is used. The lead zirconate titanate (PZT) has an advantage that the lead zirconate titanate has high machinability, has high productivity and high electromechanical conversion efficiency, and has an excellent characteristic as a piezoelectric material. The lithium niobate single crystal (LiNbO3) of the piezoelectric single crystal is one of non-lead piezoelectric materials having a high mechanical Q value suitable for an ultrasound transducer for a high-output use. Since lead is not used, the lithium niobate single crystal (LiNbO3) of the piezoelectric single crystal is suitable for environmental properties.

In the stacked transducer 41, positive-side electrode plates 62 to be positive electrode layers and negative-side electrode plates 63 to be negative electrode layers, which are made of metal such as copper in a polygonal shape, here, a rectangular shape, are alternately interposed among an insulator 42, eight rectangular piezoelectric substances 61, and an insulator 43.

As shown in Fig. 5, the stacked transducer 41 is stacked such that four corner portions and four sides of the insulators 42 and 43, four corner portions and four sides of the eight rectangular piezoelectric substances 61, and four corner portions and four sides of the respective electrode plates 62 and 63 coincide with one another. The entire stacked transducer 41 is built in a substantially square pole shape. That is, shapes of front and rear surfaces of the respective insulators 42 and 43, the respective rectangular piezoelectric substances 61, and the respective electrode plates 62 and 63 are substantially the same rectangular shapes. Note that surface shapes of the insulators 42 and 43, the respective rectangular piezoelectric substances 61, and the respective electrode plates 62 and 63 are not limited to rectangular shapes and may be polygonal shapes. The entire stacked transducer 41 may be configured to be stacked in a polygonal prism shape. That is, each of the insulating plates 42 and 43, the rectangular piezoelectric substances 61, and the respective electrode plates 62 and 63 only has to be a polygonal shape having at least two common corner portions.

In the positive-side electrode plates 62 and the negative-side electrode plates 63, lead-out sections 62a and 63a functioning as electrodes are extended from substantially centers of one sides. The lead-out sections 62a and 63a are stacked such that a positive side and a negative side thereof are extended in separated different directions. The lead-out sections 62a and 63a are electrically connected to wires on a positive side or a negative side in the electric cable 36 shown in Fig. 1 and Fig. 2.

The case 50 comprises a substantially columnar-shaped lid 51 functioning as a pressurizing member and a case main body 52 of a bottomed cylindrical body, to one end opening portion of which the lid 51 is screwed and fastened. The lid 51 and the case main body 52 are formed of duralumin or a titanium alloy such as 64Ti.

In the lid 51, plane sections 51a for a tightening jig for tightening to the case main body 52 are shaped in positions symmetrical with respect to a center point of an outer circumferential section of the lid 51. In the lid 51, a female screw hole 51b, in which a male screw 38a extended from the proximal end columnar section 38 of the horn 32, is formed in one end center portion. The lid 51 comprise, at the other end portion, a male screw section 51c for screwing to the case main body 52.

In the case main body 52, a female screw section 52a, with which the male screw section 51c of the lid 51 screws, is formed in an opening portion. Two wire lead-out sections 53 and two positioning member insertion sections 54 functioning as openings are formed in an outer circumferential section of the case main body 52. The wire lead-out sections 53 and the positioning member insertion sections 54 are slits formed in a longitudinal axial direction of the case main body 52. The four wire lead-out sections 53 and positioning member insertion sections 54 in total are formed in a side circumferential section of the case main body 52. Note that the wire lead-out sections 53 and the positioning member insertion sections 54 are respectively formed in point symmetrical positions around a center axis of the case main body 52.

In the ultrasound transducer 2 configured as explained above, after the stacked transducer 41 is housed in the case main body 52, the lid 51 is screwed and fastened to the case main body 52. In the ultrasound transducer 2, the horn 32 is screwed and fastened to the lid 51. Note that, in the ultrasound transducer 2, the lid 51 of the case 50, which houses the stacked transducer 41, configures a front mass and a bottom section 55 of the case main body 52 configures a back mass.

### (Assembling method for the ultrasound transducer)

An assembling method for the ultrasound transducer 2 is explained below.

First, as shown in Fig. 6, the insulators 42 and 43, the rectangular piezoelectric substances 61, and the respective electrode plates 62 and 63 are stacked and housed on an inside of the case main body 52. At this point, the rectangular piezoelectric substances 61 and the respective electrode plates 62 and 63 are housed in positions where the rectangular piezoelectric substances 61 and the respective electrode plates 62 and 63 are not in contact with an inner wall (a sidewall) of the case main body 52. That is, a state in which only the insulators 42 and 43 are in contact with the stacked transducer 41 and the insulators 42 and 43 are not in contact with the sidewall of the case main body 52 is maintained. This is a state in which only the insulators 42 and 43 are in contact with the bottom section 55 of the case main body 52 and the lid 51. A surface in contact with the insulators 42 and 43 is only a surface in a stacking direction (a vibrating direction of the stacked transducer 41). The lead-out sections 62a and 63a of the respective electrode plates 62 and 63 are disposed to be led out from the wire lead-out sections 53 formed in the case main body 52.

Subsequently, two positioning members 100 T-shaped in section having V grooves 101 formed at distal ends are inserted into the positioning member insertion sections 54 formed in the case main body 52. As shown in Fig. 7, the V grooves 101 of the positioning members 100 are brought into contact with separated corner portions located on a diagonal line of the stacked body in which the insulators 42 and 43, the rectangular piezoelectric substances 61, and the respective electrode plates 62 and 63 are stacked. The stacked body is accurately positioned in a desired position. Note that length in a height direction of the positioning members 100 is set substantially the same as or slightly shorter than a height dimension of the stacked body in which the insulators 42 and 43, the rectangular piezoelectric substances 61, and the respective electrode plates 62 and 63 are stacked.

In this state, the lid 51, which is a pressurizing member, is fastened to the case main body 52 by screwing. The stacked transducer 41 is pressurized together with the insulators 42 and 43 by a surface of the male screw section 51c of the lid 51 and a surface of the bottom section 55 of the case main body 52. That is, the lid 51 and the case main body 52 are fastened. The insulators 42 and 43 and the stacked transducer 41 are integrally assembled so as not to move. Thereafter, the two positioning members 100 are retracted and removed from the positioning member insertion sections 54.

In this way, in the ultrasound transducer 2 of the present embodiment, when the lid 51 and the case main body 52 are fastened, the V grooves 101 of the positioning members 100 are brought into contact with the corner portions of the stacked body formed by the insulators 42 and 43, the rectangular piezoelectric substances 61, and the respective electrode plates 62 and 63, whereby the stacked body is fixed such that a square pole shape in which respective corner portions and respective sides of the insulators 42 and 43, the rectangular piezoelectric substances 61, and the respective electrode plates 62 and 63 coincide with one another is maintained.

Consequently, the insulators 42 and 43, the rectangular piezoelectric substances 61, and the respective electrode plates 62 and 63 stacked and housed in the case main body 52 are fixed and held in a state in which the insulators 42 and 43, the rectangular piezoelectric substances 61, and the respective electrode plates 62 and 63 are formed in the square pole shape and pressurized in the case 50.

According to the above explanation, in the ultrasound transducer 2, using the plurality of rectangular piezoelectric substances 61 obtained by cutting out, in the rectangular shape, the non-lead single crystal material having heat resistance such as lead zirconate titanate (PZT) or lithium niobate (LiNbO3) having particularly poor machinability, it is possible to assemble, in the case 50, at high positioning accuracy, the stacked transducer 41 in which the plurality of rectangular piezoelectric substances 61 are stacked together with the respective insulators 42 and 43 and the respective electrode plates 62 and 63.

Productivity of the ultrasound transducer 2 is improved because encapsulation and pressurization and holding of the stacked transducer 41 in the case 50 can be simultaneously performed during the assembling. The stacked transducer 41 does not come into contact with and does not interfere with the lid 51 configuring the front mass and the bottom section 55 of the case main body 52 configuring the back mass in the case 50. Therefore, it is possible to obtain a highly efficient configuration in which deficiencies such as an output decrease, abrasion powder occurrence, and a short-circuit failure due to vibration attenuation are prevented.

### (Modification)

The wire lead-out sections 53 and the positioning member insertion sections 54 formed in the outer circumferential section of the case main body 52 of the case 50 may be configured as the same slits 56 and 57 as shown in Fig. 8 and Fig. 9.

The slits 56 shown in Fig. 8 are configured by integrating the wire lead-out sections 53 and the positioning member insertion sections 54 and formed to near the female screw section 52a formed in the opening portion of the case main body 52. On the other hand, the slits 57 shown in Fig. 9 are configured by integrating the wire lead-out sections 53 and the positioning member insertion sections 54 and formed to the opening portion of the case main body 52.

In the slits 56 and 57, when the lid 51 and the case main body 52 are fastened, as shown in Fig. 10, the lead-out sections 62a and 63a of the respective electrode plates 62 and 63 are disposed to be lead out. The two positioning members 100 brought into contact with the separated corner portions of the stacked body to be fixed and held are inserted into the slits 56 and 57. By adopting such a configuration, in the ultrasound transducer 2, a structure of the case main body 52 of the case 50 can be simplified and the productivity is improved.

### (Second Embodiment)

Next, a second embodiment of the present invention is explained below with reference to the drawings. Note that, concerning the respective components described in the first embodiment, the components having the same configurations are explained using the same reference numerals and signs and detailed explanation of the components is omitted.

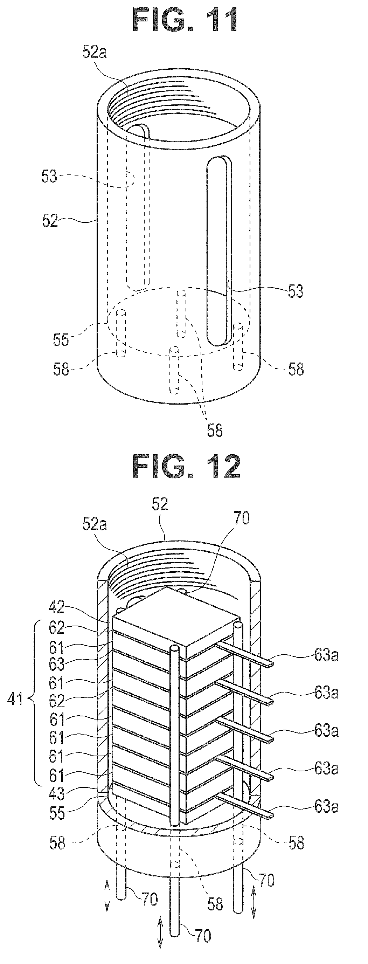

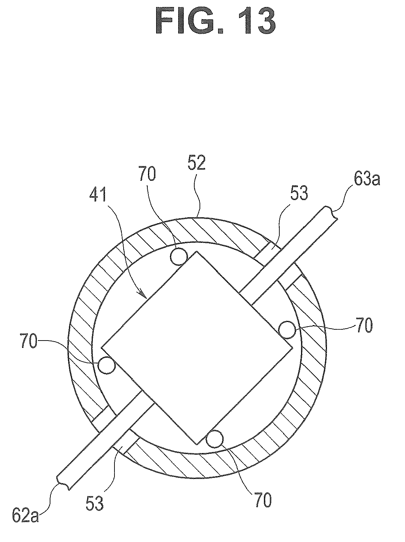

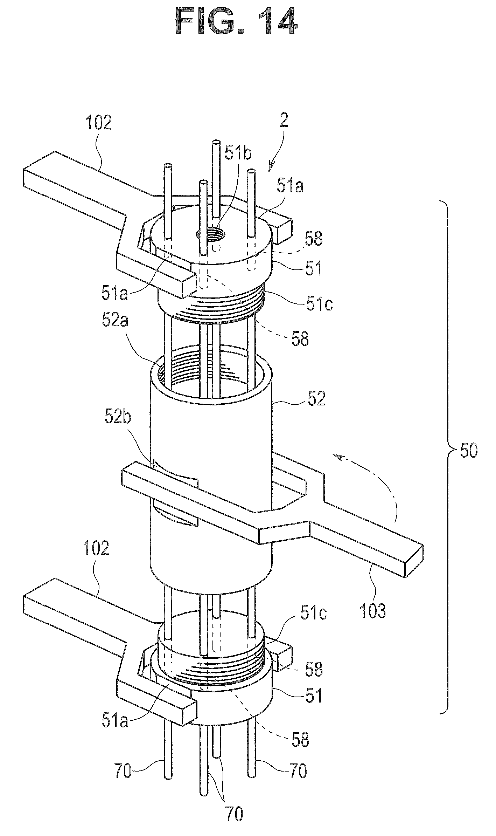

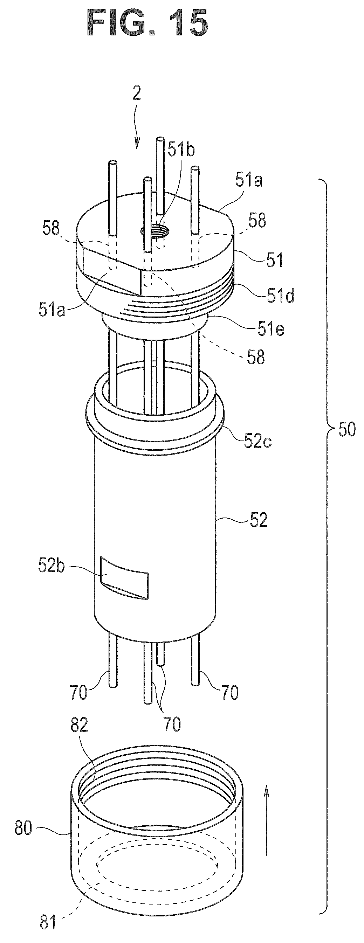

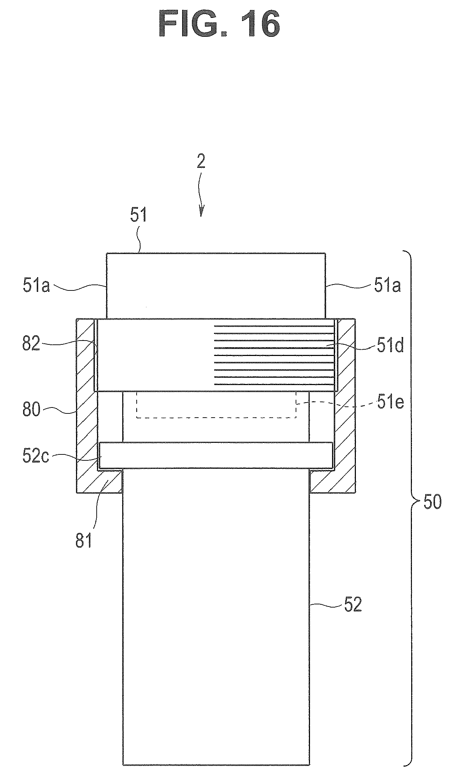

Fig. 11 is a perspective view showing a configuration of a case main body. Fig. 12 is a diagram showing a state in which a stacked transducer is housed in a case main body and held by a positioning member. Fig. 13 is a cross-sectional view showing an assembled state of an ultrasound transducer. Fig. 14 is an exploded perspective view showing an assembled state of an ultrasound transducer of a first modification. Fig. 15 is an exploded perspective view showing an assembled state of an ultrasound transducer of a second modification. Fig. 16 is a partial sectional view of the ultrasound transducer of the second modification.

In the ultrasound transducer 2 of the present embodiment, as shown in Fig. 11, at least four through holes 58 functioning as positioning member insertion sections are provided along a longitudinal direction of the case 50 in the bottom section 55 of the case main body 52 of the case 50. Note that, in the case main body 52, only the wire lead-out sections 53 are formed and the positioning member insertion sections 54 are not formed.

As shown in Fig. 12, pin-like positioning members 70 formed of metal, rigid resin, rigid rubber, a wire, a fiber, or the like are inserted into the respective four through holes 58 from an external end face side of the bottom section 55 of the case 50. As shown in Fig. 13, the four positioning members 70 are set in contact with sides in the vicinities of the respective corner portions of the insulators 42 and 43, the rectangular piezoelectric substances 61, and the respective electrode plates 62 and 63 stacked and housed in the case main body 52 and hold the insulators 42 and 43, the rectangular piezoelectric substances 61, and the respective electrode plates 62 and 63.

That is, as in the first embodiment, on the inside of the case main body 52 erected by inserting the pin-like positioning members 70 into the respective four through holes 58, in positions where the insulators 42 and 43, the rectangular piezoelectric substances 61, and the respective electrode plates 62 and 63 are not in contact with the inner wall of the case main body 52, the insulators 42 and 43, the rectangular piezoelectric substances 61, and the respective electrode plates 62 and 63 are stacked and housed in a state in which the insulators 42 and 43, the rectangular piezoelectric substances 61, and the respective electrode plates 62 and 63 are in contact with and held by the four positioning members 70. Note that the lead-out sections 62a and 63a of the respective electrode plates 62 and 63 are disposed to be led out from the wire lead-out sections 53 formed in the case main body 52.

In this state, the lid 51, which is the pressurizing member, is fastened to the case main body 52 by screwing. The stacked transducer 41 is pressurized together with the insulators 42 and 43 in the front and the back by a surface opposing the male screw section 51c of the lid 51 and a surface opposing the bottom section 55 of the case main body 52. The insulators 42 and 43 and the stacked transducer 41 are integrally assembled so as not to move. Thereafter, the four positioning members 70 are pulled out from the through holes 58 and removed.

With such a configuration as well, in the ultrasound transducer 2 of the present embodiment, using the plurality of rectangular piezoelectric substances 61 obtained by cutting out, in the rectangular shape, the non-lead single crystal material having heat resistance such as lithium niobate (LiNbO3) having poor machinability, it is possible to assemble, in the case 50, at high positioning accuracy, the stacked transducer 41 in which the plurality of rectangular piezoelectric substances 61 are stacked together with the respective insulators 42 and 43 and the respective electrode plates 62 and 63. In addition to the effects described in the first embodiment, since only the wire lead-out sections 53 are formed and the positioning member insertion sections 54 are not formed in the ultrasound transducer 2, a decrease in rigidity of the case 50 due to a plurality of slits is suppressed. A highly efficient configuration is obtained.

### (Modifications)

Note that the ultrasound transducer 2 assembled by holding the stacked body with the pin-like positioning members 70 may have a configuration described below.

### (First Modification)

In the ultrasound transducer 2 in a first modification, as shown in Fig. 14, the case 50 includes two lids 51 screwed and fastened to both ends of the cylindrical case main body 52. In each of the two lids 51, at least four through holes 58, into which the pin-like positioning members 70 are inserted to pierce through, are provided along the longitudinal direction of the case 50.

That is, as explained above, the four positioning members 70 are inserted into the through holes 58 of the two lids 51 to come into contact with sides in the vicinities of the respective corner portions of the insulators 42 and 43, the rectangular piezoelectric substances 61, and the respective electrode plates 62 and 63, which are stacked and housed in the case main body 52, and position and hold the insulators 42 and 43, the rectangular piezoelectric substances 61, and the respective electrode plates 62 and 63. Note that, in this modification, not-shown wire lead-out sections that lead out the lead-out sections 62a and 63a of the respective electrode plates 62 and 63 to the outside are provided on the lid 51 side on a rear side of the ultrasound transducer 2.

A screwing direction of one of the two lids 51 to the case main body 52 is set as a right-hand rotation direction and the screwing direction of the other is set as a left-handed rotation direction. Consequently, the case 50 is formed in a so-called turnbuckle structure in which the lids 51 are simultaneously screwed and fastened to both end opening portions of the case main body 52 by rotating only the case main body 52 in one direction with a tightening jig 103 while holding the two lids 51 with fixing jigs 102 and not rotating the lids 51. Note that, in the case 50, plane sections 52b for the tightening jig 103 are formed.

Consequently, the lids 51 do not rotate when the two lids 51 and the case main body 52 are fastened. Therefore, it is possible to prevent torque from occurring in the insulators 42 and 43, the rectangular piezoelectric substances 61, and the respective electrode plates 62 and 63. Consequently, torque during fastening does not occur either in the positioning members 70 that fix and hold the stacked body formed by the insulators 42 and 43, the rectangular piezoelectric substances 61, and the respective electrode plates 62 and 63.

Therefore, the stacked transducer 41 in which the plurality of rectangular piezoelectric substances 61 are stacked together with the respective insulators 42 and 43 and the respective electrode plates 62 and 63 can be more highly accurately positioned in the case 50. Note that, after the fastening of the two lids 51 and the case main body 52, the pin-like positioning members 70 are pulled out from the through holes 58, whereby the ultrasound transducer 2 is completed.

### (Second Modification)

As shown in Fig. 15 and Fig. 16, the case 50 of the ultrasound transducer 2 in a second modification is configured to connect the lid 51 and the case main body 52 using an annular member 80 externally inserted on the case main body 52.

More specifically, in the case main body 52 of the case 50, an outward flange 52c is provided in the vicinity of the opening portion. The annular member 80 of the case 50 includes an inward flange 81 externally inserted on the case main body 52 and coming into contact with the outward flange 52c of the case main body 52 and a female screw section 82 screwed and fastened to the male screw section 51c formed in the lid 51.

The lid 51 of the case 50 includes a projecting section 51e formed to project from the male screw section 51 c, housed on the inside from the opening portion of the case main body 52, and pressurizing the stacked body formed by the insulators 42 and 43, the rectangular piezoelectric substances 61, and the respective electrode plates 62 and 63.

Note that, in the lid 51, at least four through holes 58, into which the pin-like positioning members 70 are inserted to pierce through, are provided along the longitudinal direction of the case 50. As explained above, the four positioning members 70 are inserted into the through holes 58 of the lid 51 and the case main body 52 to come into contact with sides in the vicinities of the respective corner portions of the insulators 42 and 43, the rectangular piezoelectric substances 61, and the respective electrode plates 62 and 63, which are stacked and housed in the case main body 52, and position and hold the insulators 42 and 43, the rectangular piezoelectric substances 61, and the respective electrode plates 62 and 63.

In this way, in the ultrasound transducer 2, after the opening portion of the case main body 52 is covered with the lid 51, the annular member 80 is externally inserted on the case main body 52 and screwed and fastened to the lid 51. At this point, in a state in which the outward flange 52c of the case main body 52 is in contact with the inward flange 81 of the annular member 80, the annular member 80 is screwed and fastened to the lid 51. The lid 51 and the case main body 52 are connected and fixed.

Note that, in a state in which the lid 51 and the case main body 52 are fixed and held by the aforementioned fixing jigs 102 so as not to rotate, only the annular member 80 is rotated and the lid 51 and the case main body 52 are connected and fixed. Consequently, the case 50 has a structure in which the lid 51 and the case main body 52 can be connected and fixed without rotating.

In this modification as well, not-shown wire lead-out sections that lead out the lead-out sections 62a and 63a of the respective electrode plates 62 and 63 to the outside are provided in the case main body 52 or the lid 51.

In this modification configured in this way as well, the lid 51 and the case main body 52 do not rotate when the lid 51 and the case main body 52 are connected and fixed. Therefore, it is possible to prevent torque from occurring in the insulators 42 and 43, the rectangular piezoelectric substances 61, and the respective electrode plates 62 and 63. Consequently, torque during fastening does not occur either in the positioning members 70 that fix and hold the stacked body formed by the insulators 42 and 43, the rectangular piezoelectric substances 61, and the respective electrode plates 62 and 63.

Therefore, in this modification as well, the stacked transducer 41 in which the plurality of rectangular piezoelectric substances 61 are stacked together with the respective insulators 42 and 43 and the respective electrode plates 62 and 63 can be more highly accurately positioned in the case 50. Note that, after the lid 51 and the case main body 52 are connected and fixed by the annular member 80, the pin-like positioning members 70 are pulled out from the through holes 58, whereby the ultrasound transducer 2 is completed.

Note that, in the respective configurations explained above, the pin-like positioning members 70 are pulled out after the assembly process of the ultrasound transducer 2. However, when a material less easily hindering vibration such as resin, rubber, a wire, or a fiber is used, the pin-like positioning members 70 may be left without being pulled out or only portions extending from the case main body 52 or the lid 51 may be cut to complete the ultrasound transducer 2.

### (Third Embodiment)

Next, a third embodiment of the present invention is explained below with reference to the drawings. Note that a configuration of the ultrasound transducer 2 of the present embodiment is a modification of the first embodiment. Concerning the respective components described in the first embodiment, the components having the same configurations are explained using the same reference numerals and signs and detailed explanation of the components is omitted.

In the present embodiment, concerning the configuration of the ultrasound transducer 2 described in the first embodiment, a configuration for solving problems caused by tolerances in a thickness direction during manufacturing of the two insulators 42 and 43 and the plurality of rectangular piezoelectric substances 61 and the plurality of electrode plates 62 and 63 configuring the stacked transducer 41 is explained below.

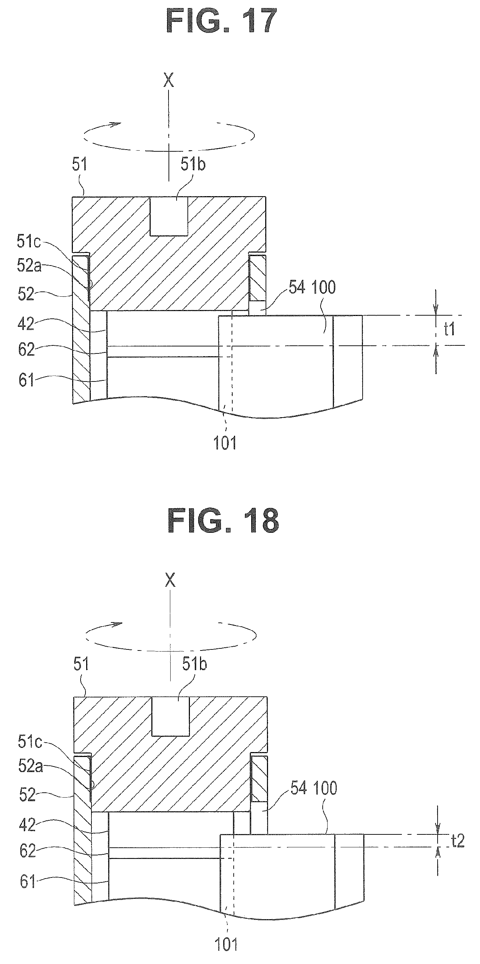

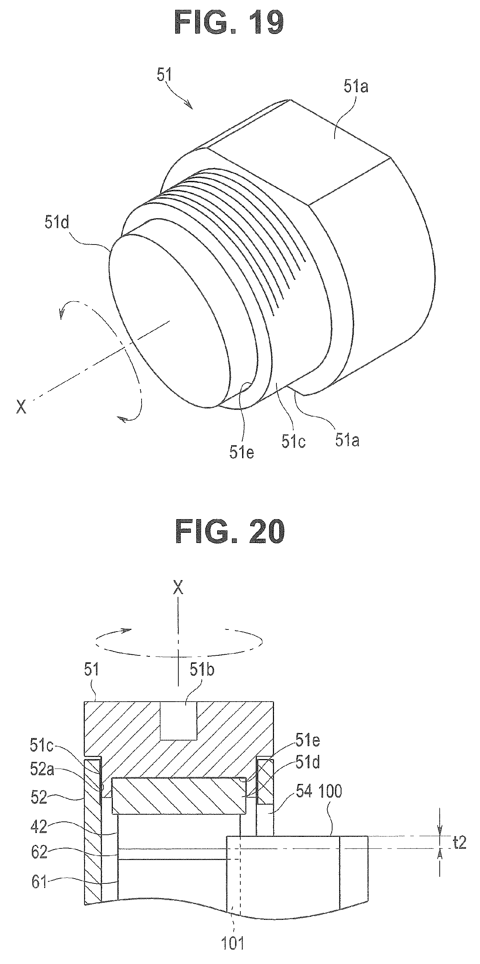

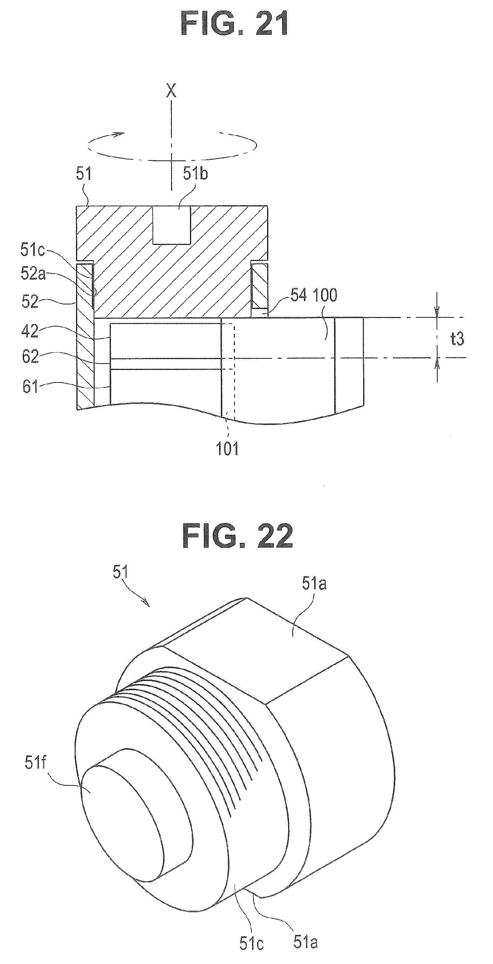

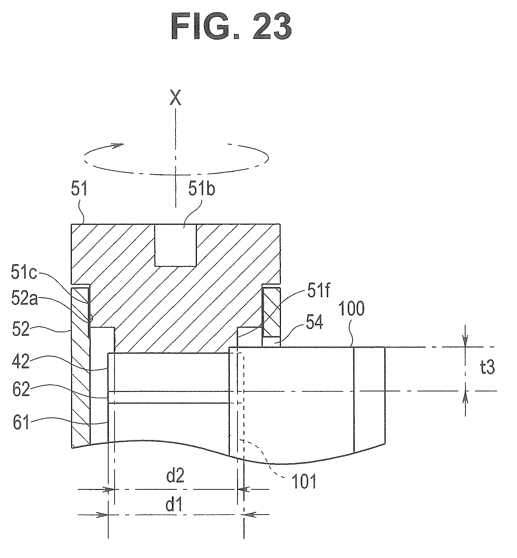

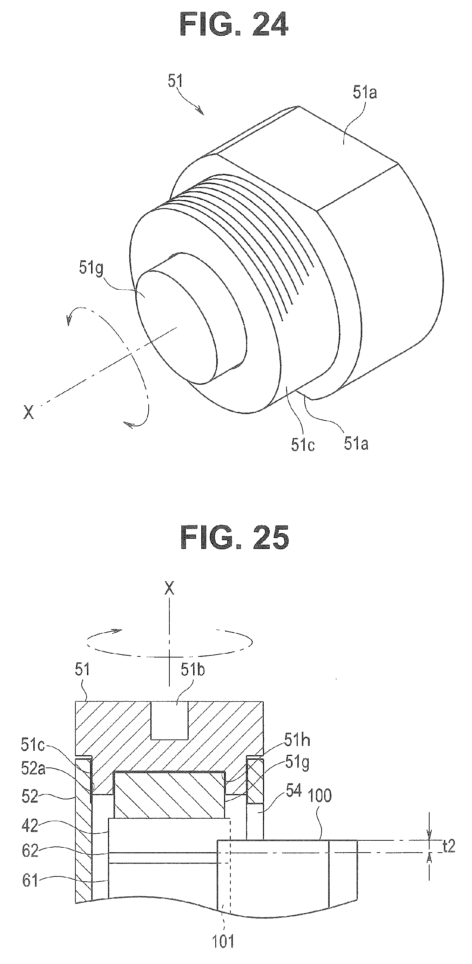

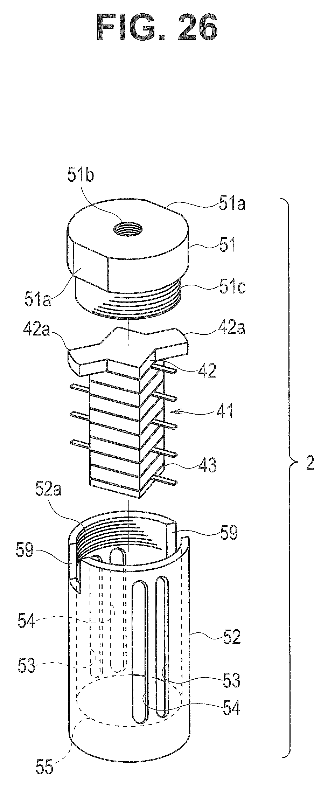

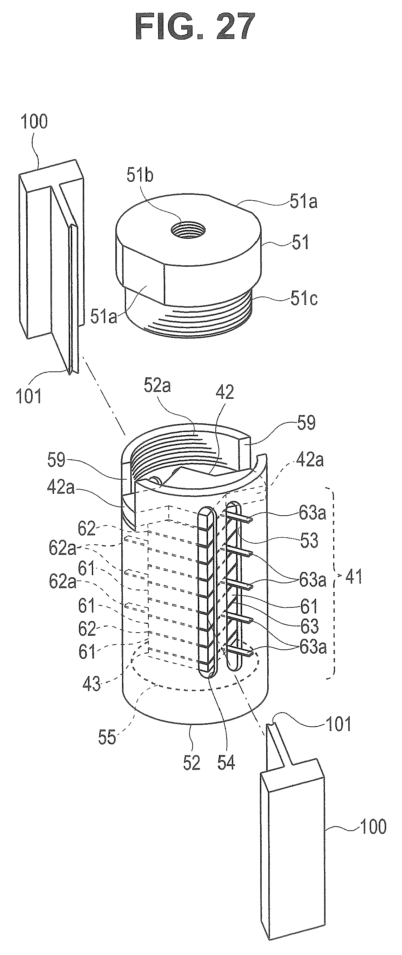

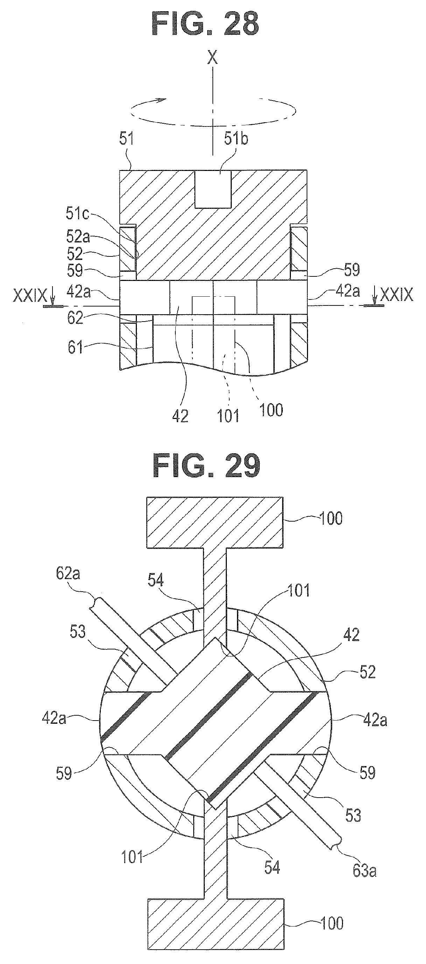

Note that Fig. 17 is a partial sectional view of a state in which a stacked body formed by insulators, rectangular piezoelectric substances, and electrode plates is positioned by positioning members in a case and pressurized by a lid. Fig. 18 is a partial sectional view of a state in which the stacked body having tolerances of plus tendency in the insulators, the rectangular piezoelectric substances, and the electrode plates is positioned by the positioning members in the case and pressurized by the lid. Fig. 19 is a perspective view showing a configuration of a lid according to a first aspect. Fig. 20 is a partial sectional view of a state in which a stacked body formed by insulators, rectangular piezoelectric substances, and electrode plates is positioned by positioning members in a case and pressurized by a lid according to the first aspect. Fig. 21 is a partial sectional view of a state in which the stacked body having tolerances of minus tendency in the insulators, the rectangular piezoelectric substances, and the electrode plates is positioned by the positioning members in the case and pressurized by the lid. Fig. 22 is a perspective view showing a configuration of a lid according to a second aspect. Fig. 23 is a partial sectional view of a state in which a stacked body formed by insulators, rectangular piezoelectric substances, and electrode plates is positioned by positioning members in a case and pressurized by a lid according to the second aspect. Fig. 24 is a perspective view showing a configuration of a lid according to a third aspect. Fig. 25 is a partial sectional view of a state in which a stacked body formed by insulators, rectangular piezoelectric substances, and electrode plates is positioned by positioning members in a case and pressurized by a lid according to the third aspect. Fig. 26 is an exploded perspective view showing a configuration of an ultrasound transducer according to a fourth aspect. Fig. 27 is an exploded perspective view showing an assembled state of the ultrasound transducer according to the fourth aspect. Fig. 28 is a partial sectional view of a state in which a stacked body formed by insulators, rectangular piezoelectric substances, and electrode plates is positioned by positioning members in a case and pressurized by a lid according to the fourth aspect. Fig. 29 is a cross-sectional view of the ultrasound transducer taken along line XXIX-XXIX in Fig. 28.

In the ultrasound transducer 2, it is desirable for stability of holding that, when the lid 51, which is the pressurizing member, is screwed and fastened to the case main body 52, the V grooves 101 of the positioning members 100 come into contact with the stacked transducer 41 formed by the two insulators 42 and 43, the plurality of rectangular piezoelectric substances 61, and the plurality of electrode plates 62 and 63 in a substantially entire area in the stacking direction to position the stacked transducer 41.

That is, in the ultrasound transducer 2, it is desirable that, when the lid 51, which is the pressurizing member, is screwed and fastened to the case main body 52, in a state in which the V grooves 101 of the positioning members 100 are in contact with the two insulators 42 and 43, the plurality of rectangular piezoelectric substances 61, and the plurality of electrode plates 62 and 63 in the height direction thereof, as shown in Fig. 17, in particular, the V grooves 101 of the positioning members 100 are in contact with and hold the stacked transducer 41 at sufficient predetermined length t1 in the height direction of the insulator 42 that is in contact with the lid 51, which is the pressurizing member.

Incidentally, in a structure in which height of the V grooves 101 of the positioning members 100 is set larger than or equal to entire height of the stacked transducer 41, which is the stacked body in which the plurality of rectangular piezoelectric substances 61 and the plurality of electrode plates 62 and 63 are stacked, and the two insulators 42 and 43 housed in the case 50 and the stacked transducer 41 is held in the entire stacking direction, the lid 51 interferes with the positioning members 100 during pressurizing and fastening by tightening of the lid 51, which is the pressurizing member, to the case main body 52 and the pressurization and the positioning cannot be performed. Therefore, the height of the positioning members 100 needs to be designed to be smaller than the height of the stacked transducer 41.

However, in the two insulators 42 and 43 and the stacked transducer 41, even if a height (thickness) dimension of the plurality of rectangular piezoelectric substances 61 and the plurality of electrode plates 62 and 63 is designed in a predetermined specified value, actually, tolerances during manufacturing are present in a dimension in the height direction.

As an example, when thickness of the insulators 42 and 43 formed of aluminum is set to 0.5 mm, thickness of the rectangular piezoelectric substances 61 formed of lithium niobate single crystal (LiNbO3) is set to 0.5 mm, and thickness of the electrode plates 62 and 63 formed of copper is set to 0.1 mm, for example, it is likely that tolerances in the height (thickness) direction due to machining may occur by approximately 0.05 mm at most in the respective heights.

In the present embodiment, the two insulators 42 and 43, the eight rectangular piezoelectric substances 61, and the nine electrode plates 62 and 63 are stacked in the stacked body. Therefore, an error that could occur in a height position where the insulator 42 in contact with the lid 51 is set in the case main body 52 is integration of the tolerances, that is, integration of the tolerance that occurs in the insulator 43 that is in contact with the bottom section 55 of the case main body 52, the tolerance that occurs in the eight rectangular piezoelectric substances 61, and the tolerance that occurs in the nine electrode plates 62 and 63. The error is ±0.9 mm [=(1+8+9)×0.05] at most.

That is, in the stacked body including the two insulators 42 and 43 and the stacked transducer 41, setting height of the insulator 42 having thickness of 0.5 mm is likely to fluctuate in a range of ±0.9 mm with respect to a design value.

A state in which all the members are finished with the maximum tolerances on a minus side or a plus side is less likely to occur. However, a minor state could easily occur.

More specifically, the two insulators 42 and 43, the plurality of rectangular piezoelectric substances 61, or the plurality of electrode plates 62 and 63 are machined by batch treatment for cutting out a plurality of pieces from respective one members, for example, wafers, plate materials, or the like of aluminum, lithium niobate single crystal (LiNbO3), or copper.

Therefore, a situation occurs in which tolerances of plus tendency or minus tendency uniformly occur in all of the two insulators 42 and 43, the plurality of rectangular piezoelectric substances 61, or the plurality of electrode plates 62 and 63.

When the tolerances of the plus tendency occur respectively in the two insulators 42 and 43, the plurality of rectangular piezoelectric substances 61, or the plurality of electrode plates 62 and 63, for example, as shown in Fig. 18, because of tolerance integration of the stacked transducer 41, predetermined length t2 at which the V grooves 101 of the positioning members 100 are in contact with the insulator 42 that is in contact with the lid 51, which is the pressurizing member, is smaller than the predetermined length t1 described above (see Fig. 17) (t2<t1).

When the lid 51 is pressurized and fastened to the case main body 52 in this state, a range in which the V grooves 101 of the positioning members 100 are in contact with the insulator 42 that is in contact with the lid 51 decreases. The insulator 42 receives torque from the lid 51 in a narrow (small) holding area by the V grooves 101 of the positioning members 100.

Consequently, deformation, a crack, and the like of the positioning members 100 and the insulator 42 may occur because of stress concentration due to the torque from the lid 51 to the insulator 42. As a result, it is likely that a part of the stacked transducer 41 is assembled in a state including a rotational shift in a fastening direction.

### (First aspect)

Therefore, in the ultrasound transducer 2 in a first aspect, as shown in Fig. 19 and Fig. 20, as a pressurizing section in which the lid 51, which is the pressurizing member, comes into contact with the insulator 42 and pressurizes the two insulators 42 and 43 and the stacked transducer 41 housed in the case main body 52 in the stacking direction, a disk-like rotation buffer plate 51d turnable around a center axis X with respect to the male screw section 51c is provided.

Note that, in the lid 51, a recessed groove section 51e circular in section is formed from an end face side of the male screw section 51c. The rotation buffer plate 51d is turnably engaged in the groove section 51e.

By adopting such a configuration, in a manufacturing process of the ultrasound transducer 2, torque generated when the lid 51 is rotated around the center axis X and screwed and fastened to the case main body 52 is less easily transmitted to the insulator 42 that is in contact with the rotation buffer plate 51d.

That is, since a contact surface with the recessed section 51 e of the lid 51 slips, the rotation buffer plate 51d in contact with the insulator 42 can buffer generation of the rotation torque to the insulator 42.

Therefore, large pressure applied to the V grooves 101 of the positioning members 100 irrespective of a holding area for holding the insulator 42 is reduced.

As a result, even if dimension fluctuation, here, tolerances of the plus tendency occur during manufacturing of the two insulators 42 and 43 and the plurality of rectangular piezoelectric substances 61 or the plurality of electrode plates 62 and 63 configuring the stacked transducer 41, the V grooves 101 of the positioning members 100 come into contact therewith at the short predetermined length t2, and the holding area for holding the insulator 42 decreases, deformation, a crack, and the like of the positioning members 100 and the insulator 42 and a positional shift and the like of the insulator 42 less easily occur.

That is, even if thickness of the two insulators 42 and 43, the plurality of rectangular piezoelectric substances 61, or the plurality of electrode plates 62 and 63 stacked in the case 50 deviates from a design median, in particular, it is possible to pressurize and fasten the stacked transducer 41 in a correct position of the case 50 without causing breakage, a crack, and the like in the insulator 42.

In the rotation buffer plate 51d in this aspect, it is sufficient that deformation does not occur with respect to strength necessary for pressurizing the stacked transducer 41 housed in the case main body 52. The rotation buffer plate 51d is desirably formed of a metal material such as titanium, duralumin, or stainless steel or a ceramics material such as alumina or zirconium.

Further, front and rear surfaces of the rotation buffer plate 51d are desirably formed as a mirror surface having surface roughness Ra of 0.05 or less or set in a state close to the mirror surface in order to reduce friction with the recessed section 51e of the lid 51 and the insulator 42. Note that, in order to further reduce friction of contact surfaces between the rotation buffer plate 51d and the recessed section 51e of the lid 51 and the insulator 42, grease or the like may be applied to interfaces of the respective contact surfaces.

As described above, in the ultrasound transducer 2, even if a decrease in the holding area of the insulator 42 by the positioning members 100 involved in the dimension fluctuation of the stacked transducer 41, here, the tolerance of the plus tendency occurs, the torque in screwing and fastening the lid 51 to the case main body 52 is less easily transmitted to the insulator 42. Therefore, deformation of the positioning members 100 and a positional shift, a crack, and the like of the insulator 42 less easily occur. The stacked transducer 41 does not cause a rotational shift in the fastening direction of the lid 51. It is possible to assemble the two insulators 42 and 43 and the stacked transducer 41 to predetermined positions of the case 50.

Incidentally, contrary to the above description, when the dimension fluctuation of the stacked body including the insulators 42 and 43 and the stacked transducer 41 is, for example, a tolerance of the minus tendency, because of tolerance integration, a height dimension of the stacked body formed by the two insulators 42 and 43 and the stacked transducer 41 is in a lower position than a height dimension of the positioning members 100. As shown in Fig. 21, the positioning members 100 interfere with the lid 51, which is the pressurizing member. The stacked body housed in the case main body 52 cannot be pressurized. It is impossible to assemble the ultrasound transducer 2.

That is, tolerances of the minus tendency respectively occur in the two insulators 42 and 43, the plurality of rectangular piezoelectric substances 61, or the plurality of electrode plates 62 and 63. Therefore, because of tolerance integration of the stacked transducer 41, a state occurs in which one end faces of the positioning members 100 exceed the thickness of the insulator 42 and have predetermined length t3 larger than the predetermined length t1 described above (see Fig. 17) from the electrode plate 62 that is in contact with the insulator 42.

When the lid 51 is pressurized and fastened to the case main body 52 in this state, a state occurs in which the lid 51 comes into contact with the end faces of the positioning members 100 and the two insulators 42 and 43 and the stacked transducer 41 cannot be pressurized.

Consequently, the two insulators 42 and 43 and the stacked transducer 41 cannot receive a pressurizing force from the lid 51. As a result, a state occurs in which the two insulators 42 and 43, the plurality of rectangular piezoelectric substances 61, or the plurality of electrode plates 62 and 63 easily shift without being fixed in the case 50.

### (Second aspect)

Therefore, the ultrasound transducer 2 in a second aspect includes, as shown in Fig. 22 and Fig. 23, as a pressurizing section in which the lid 51, which is the pressurizing member, comes into contact with the insulator 42 and pressurizes the two insulators 42 and 43 and the stacked transducer 41 housed in the case main body 52 in the stacking direction, a columnar projecting section 51f projecting from an end face of the male screw section 51c.

The projecting section 51f is formed in a columnar shape having a diameter d2 smaller than a diameter d1 of the insulator 42 that is in contact with the projecting section 51f.

Consequently, when the lid 51, which is the pressurizing member, is screwed and fastened to the case main body 52, the projecting section 51f comes into contact with only the insulator 42 and pressurizes the two insulators 42 and 43 and the stacked transducer 41. Therefore, the lid 51 does not interfere with the positioning members 100.

Note that, taking into account tolerances of the plus tendency that occur in the two insulators 42 and 43 and the plurality of rectangular piezoelectric substances 61 and the plurality of electrode plates 62 and 63 configuring the stacked transducer 41, a dimension in the height direction (the stacking direction) of the positioning members 100 is desirably set to be equal to or larger than a maximum dimension in the height direction of the two insulators 42 and 43 and the entire stacked transducer 41.

Therefore, the ultrasound transducer 2 is configured to be capable of coping with tolerances of the minus tendency and cope with tolerances of the plus tendency as dimension fluctuation of the two insulators 42 and 43 and the stacked transducer 41. Even if a dimension in the height direction of the stacked body formed by the two insulators 42 and 43 and the stacked transducer 41 fluctuates, the insulators 42 and 43 and the stacked transducer 41 are always in contact with the V grooves 101 of the positioning members 100 and held. Therefore, it is possible to surely hold the two insulators 42 and 43 and the stacked transducer 41 by the positioning members 100 with a fixed holding force.

That is, in the ultrasound transducer 2 in this aspect, there is no fluctuation in the holding area of the insulator 42 by the positioning members 100. The positioning members 100 can secure a fixed holding area over an entire region in the stacking direction of the two insulators 42 and 43 and the stacked transducer 41. Therefore, large pressure is not locally applied and a concentrated load is not applied to the positioning members 100 and the insulator 42. Deformation of the positioning members 100 and a positional shift, a crack, and the like of the insulator 42 less easily occur. The stacked transducer 41 does not cause a rotational shift in the fastening direction of the lid 51. It is possible to assemble the two insulators 42 and 43 and the stacked transducer 41 to predetermined positions of the case 50.

### (Third aspect)

Note that, in the ultrasound transducer 2, as a configuration capable of coping with dimension fluctuation in the height direction of the two insulators 42 and 43 and the stacked transducer 41 due to tolerances of the plus tendency and the minus tendency, as shown in Fig. 24 and Fig. 25, as a configuration in which the first form and the second form are combined, a rotation buffer projecting section 51 g turnable around the center axis X may be provided that comes into contact with the insulator 42 and pressurizes the stacked transducer 41 housed in the case main body 52 in the stacking direction.

In the lid 51 in this aspect as well, a recessed groove section 51h circular in section is formed from the end face side of the male screw section 51 c. The rotation buffer projecting section 51g is turnably engaged in the groove section 51h.

With such a configuration, the ultrasound transducer 2 has action and effects same as the action and effects of the first form and the second form. Deformation of the positioning members 100 and a positional shift, a crack, and the like of the insulator 42 less easily occur. The stacked transducer 41 does not cause a rotational shift in the fastening direction. It is possible to assemble the stacked transducer 41 to a predetermined position of the case 50.

### (Fourth aspect)

In the ultrasound transducer 2 in a fourth aspect, as shown in Fig. 26 and Fig. 27, two rotation preventing protrusion sections 42a integrally formed in, for example, separating directions are extended from the insulator 42 that is in contact with the lid 51, which is the pressurizing member. Two rotation preventing groove sections 59, in which the two rotation preventing protrusion sections 42a engage, are formed in the opening portion of the case main body 52.

Not that it is desirable that a projection amount adjusted to an outer diameter of the case main body 52 is set for the two rotation preventing protrusion sections 42a and projecting end faces are formed in an arcuate shape to match an outer circumferential surface of the case main body 52.

In the case main body 52, length of the rotation preventing groove sections 59 having a margin such that the insulator 42 can freely move in the height direction along the rotation preventing groove sections 59 is set according to dimension fluctuation in the height direction of the two insulators 42 and 43 and the stacked transducer 41 due to tolerances of the plus tendency and the minus tendency.

Further, dimensions in a width direction of the rotation preventing protrusion sections 42a and the rotation preventing groove sections 59 are set to be substantially the same.

In the ultrasound transducer 2 configured in this way, as shown in Fig. 28 and Fig. 29, the respective two rotation preventing protrusion sections 42a of the insulator 42 fit in the two rotation preventing groove sections 59 of the case main body 52. Consequently, rotation of the insulator 42 around the center axis of the case main body 52 is restricted.

Therefore, when the lid 51 is screwed and fastened to the case main body 52, even if the lid 51 comes into contact with the insulator 42 and torque is given to the insulator 42, the insulator 42 comes into contact with wall surfaces of the rotation preventing groove sections 59, does not move around the center axis of the case main body 52, and can move only in the height direction (the pressurizing direction).

Consequently, the torque from the lid 51 is not transmitted to the stacked transducer 41 and the positioning members 100 via the insulator 42. The stacked transducer 41 positioned by the positioning members 100 does not cause a rotational shift in the fastening direction of the lid 51. It is possible to assemble the stacked transducer 41 to a predetermined position of the case 50.

In order to cause a resultant force of torque, which acts on the case main body 52 from the lid 51 through the insulator 42 when the lid 51 is screwed and fastened to the case main body 52, to act on the case main body 52 as a couple of forces, the rotation preventing protrusion sections 42a of the insulator 42 are desirably provided in at least two places at equal intervals in a circumferential direction.

Further, in terms of distributing torque that occurs when the lid 51 is screwed and fastened to the case main body 52, the rotation preventing protrusion sections 42a of the insulator 42 are desirably provided in plurality.

Note that the rotation preventing groove sections 59 provided in the case main body 52 may also be used as the wire lead-out sections 53 or the positioning member insertion sections 54.

In the ultrasound transducer 2 configured as explained above, torque at time when the lid 51 is screwed and fastened to the case main body 52 is not given to the positioning members 100 via the insulator 42. Therefore, the ultrasound transducer 2 has action and effects same as the action and effects of the first aspect to the third aspect. Deformation of the positioning members 100 and a positional shift, a crack, and the like of the insulator 42 less easily occur. The stacked transducer 41 does not cause a rotational shift in the fastening direction. It is possible to assemble the stacked body formed by the two insulators 42 and 43 and the stacked transducer 41 to a predetermined position of the case 50.

The invention described in the aforementioned embodiments is not limited to the embodiments and the modifications. Besides, in an implementation stage, various modifications can be implemented in a range not departing from the spirit of the invention. Further, inventions in various stages are included in the embodiments. Various inventions can be extracted according to appropriate combinations in a disclosed plurality of constituent elements.

For example, when the described problems can be solved and the described effects can be obtained even if several constituent elements are deleted from all the constituent elements described in the embodiments, a configuration in which the constituent elements are deleted can be extracted as an invention.

This application is based upon and claims the benefit of priority from Japanese Patent Application No. 2013-139890 filed in Japan on July 3, 2013 and the contents described above are incorporated in the specification, the claims, and the drawings of Japanese Patent Application No. 2013-139890.

## Claims

1. An ultrasound vibration device comprising:
a stacked transducer in which a plurality of piezoelectric substances and a plurality of electrode plates are stacked, an external shape of the stacked transducer being formed in a polygonal prism shape;
insulators disposed at both ends of the stacked transducer;
a case main body that houses the piezoelectric transducer and the insulators;
a pressurizing member screwed and fastened to an opening portion of the case main body to pressurize and fix the stacked transducer and the insulators in the case main body; and
a plurality of positioning-member insertion sections into which a positioning member is inserted, the positioning member retaining, in the case main body, the stacked transducer and the insulators in the polygonal prism shape and positioning the stacked transducer in a position where the stacked transducer is not in contact with an inner wall of the case main body and one ends of the insulators are in contact with the pressurizing member.

2. The ultrasound vibration device according to claim 1, wherein the plurality of piezoelectric substances are formed of a piezoelectric single crystal.

3. The ultrasound vibration device according to claim 1 or 2, wherein the plurality of positioning member insertion sections are a plurality of openings formed in the case main body or the pressurizing member.

4. The ultrasound vibration device according to claim 3, wherein the plurality of openings are slits formed along a longitudinal direction of the case main body.

5. The ultrasound vibration device according to claim 4, wherein
a plurality of lead-out sections connected to a wire, to which driving power from an outside is supplied, are extended from the plurality of electrode plates, and
the plurality of slits also function as wire lead-out sections that lead out the plurality of lead-out sections to an outside of the case main body.

6. The ultrasound vibration device according to any one of claims 1 to 5, wherein
the case main body is a cylindrical body and includes cases, to which the pressurizing member is screwed and fastened, respectively in both end opening portions of the case main body, and
a screwing direction of one of the pressurizing member to the case main body is set as a right-handed rotation direction and a screwing direction of another of the pressurizing member is set as a left-handed rotation direction.

7. The ultrasound vibration device according to any one of claims 1 to 5, wherein the case main body is a bottomed cylindrical body, in an outer circumferential section of which an outward flange is formed and, when an annular member including an inward flange externally inserted on the case main body and coming into contact with the outward flange is screwed and fastened to the pressurizing member, the pressurizing member is connected and fixed to the opening portion of the case main body.

8. A manufacturing method for the ultrasound vibration device according to any one of claims 1 to 5, the manufacturing method comprising:
housing the stacked transducer, at both the ends of which the insulators are disposed, in a position in non-contact with the inner wall of the case main body;
inserting a plurality of the positioning members having V grooves formed at distal ends into the plurality of positioning member insertion sections of the case main body, bringing the V grooves of the positioning members into contact with opposed corner portions of a stacked body formed by the insulator and the stacked transducer, and positioning the stacked body in a state in which the stacked body is retained in the polygonal prism shape in the case main body;
screwing and fastening the pressurizing member to the case main body and pressurizing and fixing the stacked body with the pressurizing member and a bottom section of the case main body; and
retracting and removing the plurality of positioning members from the positioning member insertion sections.

9. An ultrasound medical apparatus comprising:
the ultrasound vibration device according to any one of claims 1 to 7; and
a probe distal end portion to which ultrasound vibration generated by the ultrasound vibration device is transmitted, the probe distal end portion treating a biological tissue.
